**Europäisches Patentamt**

**European Patent Office**

**Office européen des brevets**

(19)

(11) Veröffentlichungsnummer: **0 131 540**

**A2**

(12) # EUROPÄISCHE PATENTANMELDUNG

(21) Anmeldenummer: 84730075.3

(22) Anmeldetag: 05.07.84

(51) Int. Cl.⁴: **A 61 K 49/00**

(30) Priorität: 06.07.83 DE 3324754

(43) Veröffentlichungstag der Anmeldung:
16.01.85 Patentblatt 85/3

(84) Benannte Vertragsstaaten:
AT BE CH DE FR GB IT LI LU NL SE

(71) Anmelder: SCHERING AKTIENGESELLSCHAFT Berlin
und Bergkamen
Müllerstrasse 170/178 Postfach 65 03 11
D-1000 Berlin 65(DE)

(72) Erfinder: Rasor, Julia S.
10397A Meadow Place
Cupertino California 95014(US)

(72) Erfinder: Tickner, Ernest Glenn
10050 Burchell Road
Gilroy California 95020(US)

(54) Ultraschallkontrastmittel sowie dessen Herstellung.

(57) Ultraschallkontrastmittel, enthaltend Mikropartikel von Maltose, Dextrose, Laktose oder Galaktose und Gasbläschen in einem flüssigen Träger, dadurch gekennzeichnet, daß der flüssige Träger Wasser, physiologische Elektrolytlösung wie 0,9 %ige Natriumchlorid-Lösung, Ringer-Lösung oder Tyrode-Lösung oder eine wäßrige Lösung von Maltose Dextrose, Laktose oder Galaktose darstellt. Das Ultraschallkontrastmittel ist hervorragend zur Echogebung von flüssigen Medien - besonders bei Ultraschallaufnahmen von blutdurchflossenen Geweben - geeignet.

EP 0 131 540 A2

Die Erfindung betrifft die in den Ansprüchen gekennzeichneten Gegenstände.

Die Untersuchung von Organen mit Ultraschall (Sonographie) ist eine seit einigen Jahren gut eingeführte
und praktizierte diagnostische Methode. Ultraschallwellen im Megahertz-Bereich (oberhalb 2 Mega-Hertz
mit Wellenlängen zwischen 1 und 0,2 mm) werden an Grenzflächen von unterschiedlichen Gewebearten reflektiert.
Die dadurch entstehenden Echos werden verstärkt und
sichtbar gemacht. Von besonderer Bedeutung ist dabei
die Untersuchung des Herzens mit dieser Methode, die
Echokardiographie genannt wird (Haft, J.I. et al.:
Clinical echocardiography, Futura, Mount Kisco, New York
1978; Köhler, E. Klinische Echokardiographie, Enke,
Stuttgart 1979; Stefan, G. et al.: Echokardiographie,
Thieme, Stuttgart-New York 1981; G. Biamino, L. Lange:
Echokardiographie, Hoechst AG, 1983.

Da sämtliche Flüssigkeiten - auch das Blut - keine Echos
liefern, wurde nach Möglichkeiten gesucht, das Blut
und seine Strömung für eine Ultraschall-Untersuchung
sichtbar zu machen, was durch die Zugabe von feinsten
Gasbläschen auch möglich ist.

Aus der Literatur sind mehrere Methoden zur Herstellung
der Gasbläschen bekannt. Sie lassen
sich beispielsweise vor der Injektion in den Blutstrom
durch heftiges Schütteln oder Rühren von Lösungen wie
Salzlösungen, Farbstofflösungen oder von vorher entnommenem Blut erzeugen.

Obwohl man dadurch eine Ultraschall-Kontrastgebung erreicht, sind diese Methoden mit schwerwiegenden Nachteilen verbunden, die sich in schlechter Reproduzierbarkeit, stark schwankender Größe der Gasbläschen und - bedingt durch einen Anteil an sichtbaren großen Bläschen - einem gewissen Embolie-Risiko äußern. Diese Nachteile wurden durch andere Herstellungsverfahren teilweise behoben, wie beispielsweise durch das US-Patent 3,640,271, in dem Bläschen mit reproduzierbarer Größe durch Filtration oder durch die Anwendung einer unter Gleichstrom stehenden Elektrodenvorrichtung erzeugt werden. Dem Vorteil in der Möglichkeit, Gasbläschen mit reproduzierbarer Größe herstellen zu können, steht der erhebliche technische Aufwand als Nachteil gegenüber.

In dem US-Patent 4,276,885 wird die Herstellung von Gasbläschen mit definierter Größe beschrieben, die mit einer vor dem Zusammenfließen schützenden Gelatine-Hülle umgeben sind. Die Aufbewahrung der fertigen Bläschen kann nur im "eingefrorenen" Zustand erfolgen, beispielsweise durch Aufbewahren bei Kühlschranktemperatur, wobei sie zur Verwendung wieder auf Körpertemperatur gebracht werden müssen.

In dem US-Patent 4,265,251 wird die Herstellung und Verwendung von Gasbläschen mit einer festen Hülle aus Sacchariden beschrieben, die mit einem unter Druck stehenden Gas gefüllt sein können. Stehen sie unter
Normaldruck, so können sie als Ultraschallkontrastmittel eingesetzt werden; bei Verwendung mit erhöhtem
Innendruck dienen sie der Blutdruckmessung. Obwohl
hierbei die Aufbewahrung der festen Gasbläschen kein
Problem darstellt, ist der technische Aufwand bei der
Herstellung ein erheblicher Kostenfaktor.

Die Risiken der nach dem Stand der Technik zur Verfügung
stehenden Kontrastmittel werden durch zwei Faktoren hervorgerufen: Größe und Anzahl sowohl der Feststoffpartikel
als auch der Gasbläschen.

Der bisher geschilderte Stand der Technik gestattet die
Herstellung von Ultraschallkontrastmitteln, die stets ·
nur einige der geforderten Eigenschaften besitzen:

1.) Ausschalten des Embolierisikos
     - Gasbläschen (Größe und Anzahl)
     - Feststoffpartikel (Größe und Anzahl)

2.) Reproduzierbarkeit

3.) genügend lange Stabilität

4.) Lungengängigkeit, z.B. um Ultraschall-Kontrastierung
     des linken Herzteiles zu erhalten

5.) Kapillargängigkeit

6.) Sterilität und Pyrogenfreiheit der Zubereitung

7.) leichte Herstellbarkeit mit vertretbarem Kostenaufwand

8.) und problemlose Bevorratung.

In der europäischen Patentanmeldung mit der Veröffentlichungs-Nummer 52575 wird zwar die Herstellung von Gasbläschen beschrieben, die diese erforderlichen Eigenschaften besitzen sollen. Zu ihrer Herstellung werden
Mikropartikel einer festen kristallinen Substanz,
wie beispielsweise Galaktose, in einer Trägerflüssigkeit suspendiert, wobei das Gas, das an der
Partikeloberfläche adsorbiert, in Hohlräumen zwischen
den Partikeln oder in interkristallinen Hohlräumen
eingeschlossen ist, die Gasbläschen bildet. Die so
entstandene Suspension von Gasbläschen und Mikropartikel kann innerhalb von 10 Minuten injiziert
werden. Der flüssige Träger transportiert und dispergiert die Mikropartikel und Gasbläschen und muß
physiologisch verträglich sein.

In der europäischen Patentanmeldung Nr.52575 werden als
Trägerlösungen wässrige Lösungen als besonders geeignet
beschrieben, da deren Verträglichkeit unproblematisch
ist. Es werden jedoch Zusätze benötigt, die die Viskosität der Lösung erhöhen. Als dafür besonders geeignet
werden Glycerin, Propylenglykol, Polyethylenglykol
300 und 400, Polyvinylpyrrolidon und andere mit Wasser
mischbare oder in Wasser dispergierbare Polymere genannt, soweit sie genügend verträglich sind. Als besonders
bevorzugt wird im Beispiel 4 dieser Anmeldung ein
flüssiger Träger beschrieben, der aus einer wässrigen
5%igen Dextrose-Lösung besteht, die noch 10 % Propylenglykol enthält.

- 6 -

Die Verwendung von Propylenglykol bei den mit diesen
Ultraschall-Kontrastmitteln durchgeführten Untersuchungen
ist allerdings nicht unproblematisch, wenn man berücksichtigt, daß diagnostische Untersuchungen des Herzens
in erster Linie bei Risikopatienten durchgeführt werden,
bei denen jeglicher problembehaftete Stoff nach Möglichkeit vermieden werden soll. Nach den Untersuchungen von
P.P, Singh u.a., Arzneimittel-Forschung/Drug Research
32 (II.) Nr. 11/1982, S. 1443 ff hat sich gezeigt,
daß Propylenglykol in höheren Konzentrationsbereichen
unterschiedliche neuropsychopharmakologische Wirkungen
bei Mäusen und Ratten hervorruft. Andere Autoren berichten über Blutdruckbeeinflussung (Budden, R. u.a.
Arzneim.-Forschung 28/9: 1586 (1978).

Im Hinblick darauf, daß

1.) Ultraschall-Untersuchungen mit Kontrastmitteln mehr-
    fach in kurzen Abständen erforderlich sein können
    und daß

2.) die verwendeten Ultraschall-Kontrastmittel als
    Bolus-Injektion durchgeführt werden, um den
    Verdünnungseffekt durch das strömende Blut so weit
    wie möglich entgegenzuwirken,

kann nicht ausgeschlossen werden, daß die das Gehirn erreichenden Propylenglykol-Konzentrationen - besonders bei Risikopatienten - nicht mehr unbedenklich sind.

Es war deshalb die Aufgabe dieser Erfindung, ein Ultraschallkontrastmittel bereitzustellen, das bei mindestens gleicher Wirksamkeit in der Anfärbung des Blutes in den untersuchten Organen auch ohne Propylenglykol eingesetzt werden kann.

Dabei wurde überrschenderweise festgestellt, daß durch die Verwendung eines flüssigen Trägers, der Wasser, eine physiologische Elektrolytlösung wie 0,9 %ige wäßrige Natriumchloridlösung, Ringer-Lösung oder Tyrode-Lösung oder eine wäßrige Lösung von Maltose, Dextrose, Laktose oder Galaktose ist, ohne Zusatz von viskositätserhöhenden .Stoffen, wie beispielsweise Propylenglykol, eine gleichgute bis bessere Qualität der Anfärbung bei Ultraschallaufnahmen von blutdurchströmten Organen (Herz, Adern) erreicht wird.

Zur Herstellung des erfindungsgemäßen Mittels geht man so vor, daß man die nach dem in der europäischen Patentanmeldung Nr. 52 575 beschriebenen Verfahren hergestellten Mikropartikel aus Maltose, Dextrose, Laktose oder Galaktose, wobei Galaktose bevorzugt ist, in einer wäßrigen Lösung von Maltose, Dextrose, Laktose oder vorzugsweise Galaktose unter sterilen Bedingungen suspendiert.

Wird die wäßrige Lösung von Maltose, Dextrose, Laktose oder Galaktose als flüssiger Träger verwendet, beträgt die Konzentration bis zu 30 % (Gewichtsprozent), wobei die Konzentration von der Löslichkeit des verwendeten Zuckers in Wasser abhängt. Beispielsweise verwendet man im Falle von Laktose eine bis zu 25 %ige
(Gewichtsprozent) wäßrige Lösung und im Falle von
Galaktose eine bis zu 30 %ige Lösung, wobei eine
20 %ige wäßrige Lösung bevorzugt ist.

Ein weiteres Verfahren zur Herstellung besteht darin,
die Mikropartikel in Wasser, in physiologischer Elektrolytlösung wie 0,9 %iger wäßriger Natriumchloridlösung, Ringer-Lösung oder Tyrode-Lösung zu suspendieren, wobei sich ein Teil der Mikropartikel, besonders rasch die Mikropartikel mit den geringsten Abmessungen spontan löst.

Dieses Verfahren bietet gegenüber dem Suspendieren
der Mikropartikel in einer wäßrigen Lösung von Maltose,
Dextrose, Laktose oder Galaktose den Vorteil, daß man
zur Herstellung des erfindungsgemäßen Kontrastmittels
neben den Mikropartikeln lediglich Wasser benötigt,
das länger steril aufzubewahren ist als eine wäßrige
Lösung von Maltose, Laktose, Dextrose oder Galaktose.

Die Anwendung der erfindungsgemäßen Kontrastmittel
geschieht nach der im Beispiel 7 der genannten europäischen Patentanmeldung Nr. 52 575 geschilderten
Methode. Um die Handhabung unter sterilen Bedingungen
zu erleichtern, ist es zweckmäßig, die Mikropartikel
und den flüssigen Träger in getrennten sterilen
Gefäßen (Vials) bereitzuhalten und im Bedarfsfall
den flüssigen Träger mittels Injektionsspritze dem
entsprechenden Gefäß zu entnehmen und in das Gefäß
mit den Mikropartikel zu überführen, die Mischung
ca. 5-10 Sekunden zu schütteln, danach die benötigte
Menge der Suspension zu entnehmen und in eine periphere Vene zu injizieren, wobei 0,01 ml bis 1 ml pro
kg Körpergewicht appliziert werden. An die Kontrastmittelinjektion schließt sich sofort die Injektion von 5-10 ml Kochsalzlösung an, damit der Kontrastmittel-Bolus so weitgehend wie möglich bis zum Erreichen des Herzens erhalten bleibt. Die Ultraschall-Messung und -Untersuchung erfolgt wie in der europäischen Patentanmeldung beschrieben.

Bei den in Prozent angegebenen Konzentrationen handelt es sich stets um Gewichts-/Volumenprozente (W/V).

Beispiel 1

A) Herstellung der Trägerflüssigkeit 80 g Galactose werden in Wasser für Injektionszwecke gelöst ,bis zu einem Volumen von 400 ml aufgefüllt, durch ein 0,2 μm-Filter gedrückt, jeweils
5 ml dieses Filtrats in 5 ml-Vials abgefüllt und
20 Minuten bei 120°C sterilisiert.

B) Herstellung der gebrauchsfertigen Kontrastmittellösung:

Man gibt den Inhalt eines Vials mit Trägerflüssigkeit (5 ml) zu 2 g   · Galaktose-Mikropartikel
(hergestellt nach europäischer Patentanmeldung
52 575, Beispiel 1), die sich in einem 10 ml-Vial
befinden und schüttelt 5 bis 10 Sekunden.

Beispiel 2

A) Herstellung der Trägerflüssigkeit Wasser für Injektionszwecke wird zu jeweils 5 ml
in 5 ml-Vials abgefüllt und 20 Minuten bei 120°C
sterilisiert.

B) Herstellung der gebrauchsfertigen Kontrastmittel-
Lösung:

Man gibt den Inhalt eines Vials mit Trägerflüssigkeit (Wasser,5 ml) zu 2 g Galaktose-Mikropartikel,
die sich in einem 10 ml Vial befinden und schüttelt
5 bis 10 Sekunden.

Beispiel 3

A) Herstellung der Trägerflüssigkeit 9 g Natriumchlorid werden in Wasser für Injektionszwecke gelöst, die Lösung bis zu einem Volumen von
1000 ml aufgefüllt, durch ein 0,2 µm-Filter gedrückt,
jeweils 5 ml dieses Filtrates in 5 ml-Vials abgefüllt
und 20 Minuten bei 120 °C sterilisiert.

B) Herstellung der gebrauchsfertigen Kontrastmittellösung

Man gibt den Inhalt eines Vials mit Trägerflüssigkeit
(5 ml 0,9 %ige Natriumchloridlösung) zu 2 g Galaktose-
Mikropartikel, die sich in einem 10 ml-Vial befinden
und schüttelt 5 bis 10 Sekunden.

- 1 -

Patentansprüche:

1.) Ultraschallkontrastmittel, enthaltend Mikropartikel
von Maltose, Dextrose, Laktose oder Galaktose und,
Gasbläschen in einem flüssigen Träger, dadurch gekennzeichnet, daß der flüssige Träger Wasser, physiologische Elektrolytlösung wie 0,9 %ige Natriumchloridlösung, Ringer-Lösung oder Tyrode-Lösung
oder eine wäßrige Lösung von Maltose, Dextrose,
Laktose oder Galaktose darstellt.

2.) Ultraschallkontrastmittel gemäß Anspruch 1, dadurch
gekennzeichnet, daß es Mikropartikel aus Laktose in
bis zu 25 %iger (Gewichtsprozent) wäßriger Laktose-
Lösung enthält.

3.) Ultraschallkontrastmittel gemäß Anspruch 1, dadurch
gekennzeichnet, daß es Mikropartikel aus Galaktose
in bis zu 20 %iger wäßriger Galaktose-Lösung enthält.

4.) Ultraschallkontrastmittel gemäß Anspruch 1, dadurch
gekennzeichnet, daß es Mikropartikel aus Galaktose
in Wasser enthält.

5.) Ein Kit für die Herstellung eines Ultraschall-Kontrastmittel gemäß Anspruch 1 bis 4, bestehend aus
a) einem geeigneten Behälter mit einem Volumen von
   5 - 10 ml, versehen mit einem Verschluß, der die
   Entnahme des Inhalts unter sterilen Bedingungen
   ermöglicht, gefüllt mit 4 bis 7 ml, vorzugsweise
   6 ml, des flüssigen Trägers, der Wasser, physiologische Elektrolytlösung wie 0,9 %ige Natriumchlorid-Lösung, Ringer-Lösung oder Tyrode-Lösung,
   oder eine wäßrige Lösung von Maltose, Dextrose,
   Laktose oder Galaktose darstellt
   und

b) einem zweiten geeigneten Behälter mit einem
   Volumen von 5 - 1C ml, versehen mit einem Verschluß, der die Zugabe einer Lösung und die
   Entnahme des Inhalts unter sterilen Bedingungen
   ermöglicht, gefüllt mit 1 bis 2 g Mikropartikel
   aus Maltose, Dextrose, Laktose oder Galaktose
   mit einer durchschnittlichen Größe von 1 bis
   50 µm.

6.) Verfahren zur Herstellung eines Ultraschallkontrastmittels gemäß Anspruch 1, dadurch gekennzeichnet, daß man in an sich bekannter Weise
    Mikropartikel von Maltose, Dextrose, Laktose
    oder Galaktose entweder in Wasser, in physiologischer Elektrolytlösung wie 0,9 %iger wäßriger
    Natriumchloridlösung, Ringer-Lösung oder Tyrode-
    Lösung oder in einer wäßrigen Lösung von Maltose,
    Dextrose, Laktose oder Galaktose suspendiert.